# EUROPÄISCHE PATENTSCHRIFT

(11) **EP 0 504 706 B1**
(45) Veröffentlichungstag und Bekanntmachung des Hinweises auf die Patenterteilung: **21.06.1995**
(21) Anmeldenummer: 92104036.6
(22) Anmeldetag: 10.03.1992
(51) Int. Cl.: C01F 11/00, C07C 49/12, C07C 49/92

(54) **Neuartige Erdalkalimetall-Heptandionat-Verbindungen**
Heptandionate compounds of alkaline earth metals
Composés heptandionates des métaux terres alcalins

(30) Priorität: 18.03.1991 DE 4108731
(43) Veröffentlichungstag der Anmeldung: 23.09.1992
(73) Patentinhaber: SOLVAY BARIUM STRONTIUM GmbH, D-30173 Hannover (DE)
(72) Erfinder: Döllein, Günther, W-3000 Hannover 61 (DE)
(74) Vertreter: Lauer, Dieter, Dr.

(56) Entgegenhaltungen:
- EP-A- 0 055 459
- US-A- 3 098 863
- US-A- 4 251 233
- CHEMICAL ABSTRACTS, vol. 116, no. 6, 10. Februar 1992, Columbus, Ohio, US; abstract no. 50311M, N.P. KUZ'MINA ET AL.: 'Barium dipivaloylmethanate - starting compound for obtaining high temperature superconducting films by deposition from gas phase.' Seite 630 ;Spalte L ; and Zh. Neorg. Khim. 1991, 36(11),2739-44
- CHEMICAL ABSTRACTS, vol. 114, no. 26, 1. Juli 1991, Columbus, Ohio, US; abstract no. 258508X, M. LESKELA ET AL.: 'Thermoanalytical and mass spectrometric studies on volatile beta-diketone chelates.' Seite 786 ;Spalte L ; and Thermochim. Acta, 1991, 175(1), 91-8

## Beschreibung

Die Erfindung bezieht sich auf neuartige Erdalkalimetall-Heptandionat-Verbindungen, ein Verfahren zu ihrer Herstellung und ein Verfahren zu ihrer Anwendung zur Abscheidung Erdalkalimetall enthaltender Schichten.

Aus der wissenschaftlichen Literatur sind Erdalkalimetall-Heptandionat-Verbindungen der allgemeinen Formel (I), M(R)₂, worin M Calcium, Strontium oder Barium und R 2,6-Dimethyl-3,5-heptandionat, 2,2,6-Trimethyl-3,5-heptandionat bzw. 2,2,6,6-Tetramethyl-3,5-heptandionat bedeutet, bereits bekannt.

Es ist auch bereits bekannt, daß diese Verbindungen verdampfbar sind und deshalb zur Abscheidung von Erdalkalimetall enthaltenden Schichten, beispielsweise bei der Herstellung supraleitender, oxidkeramischer Schichten verwendet werden können. Die bislang bekannten und im Handel erhältlichen Erdalkalimetall-Heptandionat-Verbindungen weisen jedoch noch Nachteile auf. Sie zeigen beispielsweise ein ungleichmäßiges Gewichtsabnahme- bzw. Verdampfungsverhalten derart, daß die Gewichtsabnahme bzw. Verdampfungsrate von relativ hohen Anfangswerten bald auf relativ kleine Werte absinkt.

Aufgabe der Erfindung ist es, Erdalkalimetall-Heptandionat-Verbindungen mit vorteilhaften Eigenschaften, insbesondere im Hinblick auf die Anwendung in der MCOVD(metal organic chemical vapour deposition-)Methode, anzugeben. Diese Aufgabe wird durch die erfindungsgemäßen Erdalkalimetall-Heptandionat-Verbindungen gelöst.

Ein Gegenstand der vorliegenden Erfindung sind neue verdampfbare Verbindungen der allgemeinen Formel

M(R)₂ (I)

worin M Calcium, Strontium oder Barium und R 2,6-Dimethyl-3,5-heptandionat, 2,2,6-Trimethyl-3,5-heptandionat oder 2,2,6,6-Tetramethyl-3,5-heptandionat bedeutet, welche dadurch gekennzeichnet sind, daß im ¹H-NMR-Spektrum ein Signal für Wasserprotonen nicht nachweisbar ist.

Der Begriff "3,5-Heptandion" bezeichnet Verbindungen mit der Grundstruktur

C-C-C(O)-C-C(O)-C-C.

Der Begriff "3,5-Heptandionat" bezeichnet Verbindungen obiger Grundstruktur, bei welchen jedoch ein Proton abstrahiert ist und die deshalb eine negative Ladung aufweisen.

Selbst bei einem hohen Signal-Rausch-Verhältnis, beispielsweise >600, ist ein Wassersignal nicht zu identifizieren. Selbstverständlich muß man in einem wasserfreien Lösungsmittel messen, insbesondere in deuterierten Lösungsmitteln wie D₆-Dimethylsulfoxid.

In dieser Eigenschaft, daß nämlich in einer Lösung der erfindungsgemäßen Verbindungen in einem nichtwäßrigen Lösungsmittel im ¹H-NMR-Spektrum selbst bei Messung in Geräten, die nach dem FT-(Fourier-Transform-)Prinzip und hoher Spektrenakkumulation mit entsprechend hohem Signal-Rauschverhältnis der Methylprotonen arbeiten, ein Signal für Wasserprotonen nicht nachweisbar ist, unterscheiden sich die erfindungsgemäßen Erdalkalimetall-Heptandionat-Verbindungen von den bislang bekannten und in der Technik üblicherweise verwendeten Verbindungen.

Bevorzugte Verbindungen der allgemeinen Formel (I) sind solche, in welchen R 2,2,6,6-Tetramethyl-3,5-heptandionat bedeutet.

Vorzugsweise bedeutet M Barium.

Besonders bevorzugte Verbindungen sind Calcium-, Strontium- bzw. Barium-bis(2,2,6,6-tetramethyl-3,5-heptandionat), insbesondere Barium-bis(2,2,6,6-tetramethyl-3,5-heptandionat), kurz Ba(TMHD)₂ genannt.

Es ist bekannt, daß Metall-Diketonate in den verschiedenen Aggregatzuständen in monomerer, oligomerer bzw. polymerer Form vorliegen können. Die allgemeine Formel (I) soll nicht nur monomere, sondern auch oligomere bzw. polymere Formen der erfindungsgemäßen Verbindungen mit umfassen.

Eine besonders vorteilhafte Eigenschaft, in welcher sich die erfindungsgemäßen Verbindungen von bereits bekannten Verbindungen dieser Art unterscheiden, ist die gleichförmige Gewichtsabnahme bzw. Verdampfungsrate im Vakuum bei erhöhter Temperatur.

Erhitzt man beispielsweise eine Probe des erfindungsgemäßen Barium-bis(2,2,6,6-tetramethyl-3,5-heptandionats), Ba(TMHD)₂, bei vermindertem Druck in Stickstoffatmosphäre auf eine Temperatur von 200 °C und bestimmt die prozentuale Gewichtsabnahme, jeweils bezogen auf das ursprünglich eingesetzte Gewicht der Probe als 100 Gew.-%, so stellt man fest, daß das Verhältnis der prozentualen Gewichtsabnahme innerhalb der ersten Stunde nach Erreichen der Temperatur von 200 °C und innerhalb der sechsten Stunde nach Erreichen der Temperatur von 200 °C bei etwa 1,5:1 liegt. Teilt man die Meßdauer nach Erreichen der Temperatur in 6 Perioden von jeweils 1 Stunde, so liegen die Verhältnisse der prozentualen Gewichtsabnahme einer Periode verglichen mit der darauffolgenden Periode innerhalb einer Schwankungsbreite von 0,7:1 bis 1,5:1. Der absolute Wert der Gewichtsabnahme des erfindungsgemäßen Ba(TMHD)₂ beträgt während eines Zeitraumes von mindestens 6 Stunden nach Erreichen einer Temperatur von etwa 200 °C etwa (3 ± 0,5) Gew.-%, bezogen auf das Gesamtgewicht der Probe bei Versuchsbeginn.

Ebenso gleichförmig ist die Gewichtsabnahme beispielsweise der erfindungsgemäßen Verbindungen Ca(TMHD)₂ und Sr(TMHD)₂.

Erhitzt man beispielsweise eine Probe des erfindungsgemäßen Ca(TMHD)₂ bei vermindertem Druck in Stickstoffatmosphäre auf eine Temperatur von 200 °C und bestimmt die prozentuale Gewichtsabnahme, jeweils bezogen auf das ursprünglich eingesetzte Gewicht der Probe als 100 Gew.-% bei Versuchsbeginn, so stellt man fest, daß der Wert der prozentualen Gewichtsabnahme innerhalb der zweiten Stunde nach Erreichen der Temperatur von 200 °C und der Wert der Gewichtsabnahmen innerhalb der sechsten Stunde nach Erreichen der Temperatur von 200 °C ein Verhältnis von etwa 0,6:1 aufweisen. Teilt man die Meßdauer nach Erreichen der Temperatur in sechs Perioden von jeweils 1 Stunde, so liegt das Verhältnis der prozentualen Gewichtsabnahme der ersten Periode und der prozentualen Gewichtsabnahme der zweiten Periode zwar bei 5,6:1. Die Verhältnisse der prozentualen Gewichtsabnahme der zweiten, dritten, vierten und fünften Periode und der Gewichtsabnahme der jeweils darauffolgenden Periode liegen innerhalb einer vorteilhaft geringen Schwankungsbreite von etwa 0,55:1 bis 1,2:1. Der absolute Wert der Gewichtsabnahme des erfindungsgemäßen Ca(TMHD)₂ beträgt während eines Zeitraumes von mindestens 5 Stunden nach Erreichen einer Temperatur von etwa 200 °C etwa (0,56 ± 0,16) Gew.-%, bezogen auf das Gesamtgewicht der Probe.

Eine solche gleichförmige Gewichtsabnahme ist mit bekannten Heptandionat-Verbindungen nicht zu erreichen. Bei diesen wird vielmehr eine sehr starke prozentuale Gewichtsabnahme innerhalb der ersten Stunde und eine geringere Gewichtsabnahme ab der zweiten Stunde nach Erreichen der Aufheiztemperatur von 200 °C beobachtet. Vorteilhaft konstant über einen langen Zeitraum ist auch die Verdampfungsrate der erfindungsgemäßen Verbindungen. Bestimmt man beispielsweise die Verdampfungsrate der erfindungsgemäßen Verbindungen, gemessen in mg/min, bei 220 °C im Vakuum über einen Zeitraum von 6 Stunden, so stellt man fest, daß die Verdampfungsrate nach 6 Stunden noch etwa 69 % der ursprünglichen Verdampfungsrate beträgt. Zudem ist der Abfall sehr gleichförmig. Eine solch konstante Verdampfungrate ist mit bekannten Erdalkalimetall-Heptandionaten nicht erreichbar. Bei den bekannten Verbindungen wird ein viel stärkerer Abfall beobachtet und häufig auch eine nicht konstante Änderung der Verdampfungsrate. Üblicherweise nimmt die Verdampfungsrate nämlich bei bekannten Verbindungen innerhalb von 2 Stunden besonders stark ab. Eine nach 6 Stunden gemessene Verdampfungsphase von 60 % und mehr der ursprünglich gemessenen Verdampfungsrate ist ebenfalls eine Eigenschaft, in der sich die neuen Verbindungen von bekannten Verbindungen unterscheiden.

Ein weiterer Gegenstand der vorliegenden Erfindung ist ein Verfahren zur Herstellung der erfindungsgemäßen Verbindung der allgemeinen Formel (I). Dieses Verfahren ist dadurch gekennzeichnet, daß man eine Lösung oder Suspension der Verbindung des Metalles M in Wasser oder in einem wasserhaltigen, mit Wasser mischbaren Lösungsmittel mit dem Diketon versetzt, das gebildete Produkt abtrennt und im Vakuum bei einer Temperatur von maximal 60 °C trocknet, bis im ¹H-NMR-Spektrum Wasserprotonen nicht mehr nachweisbar sind.

Für die Aufnahme eines ¹H-NMR-Spektrums löst man die zu untersuchende Verbindung zweckmäßig in einem wasserfreien, insbesondere deuterierten Lösungsmittel, z.B. D₆-DMSO.

Man kann verschiedene anorganische oder organische Erdalkalimetallverbindungen als Ausgangsverbindungen einsetzen. Besonders geeignet sind Oxide und insbesondere Hydroxide, beispielsweise Calciumhydroxid, Strontiumhydroxid, Bariumhydroxid sowie entsprechende Hydrate bzw. Calciumoxid, Strontiumoxid oder Bariumoxid. Zur Herstellung von Verbindungen der allgemeinen Formel (I), in welchen M Barium bedeutet, geht man besonders zweckmäßig von Bariumhydroxid-Octahydrat aus. Selbstverständlich könnte man auch vom wasserfreien Bariumhydroxid oder Bariumhydroxid-Monohydrat oder anderen Hydraten ausgehen, ebenso von Bariumoxid.

Als Lösungsmittel verwendet man Wasser oder Gemische von Wasser mit wassermischbaren organischen Lösungsmitteln. Beispielsweise kann man Gemische von Wasser mit kurzkettigen aliphatischen Alkoholen, beispielsweise Methanol oder Ethanol, verwenden. Besonders bevorzugt verwendet man Wasser, das keine organischen Lösungsmittel enthält.

Das Molverhältnis von Erdalkalimetallverbindung zu Heptandion-Verbindung beträgt zweckmäßig 1:2 bis 1:2,05. Zweckmäßig legt man die Lösung bzw. Suspension der Erdalkaliverbindung im Lösungsmittel vor und gibt die Heptandion-Verbindung unter intensivem Rühren zu. Gewünschtenfalls kann man das Reaktionsgemisch zur Vervollständigung der Umsetzung weiterrühren, beispielsweise bis zu 24 Stunden bei erhöhter Temperatur. Für die Herstellung von Ba(TMHD)₂ hat sich eine Nachreaktionsphase unter Rühren von 8 bis 12 Stunden bei 40 bis 50 °C Temperatur in der Reaktionsmischung als vorteilhaft erwiesen.

Das Produkt kann dann bei Raumtemperatur von der überstehenden flüssigen Phase abgetrennt werden. Hierzu bedient man sich bekannter Methoden, zweckmäßig filtriert man im Wasserstrahlvakuum über eine Nutsche. Vorteilhaft wird das Produkt dann mit destilliertem Wasser nachgewaschen und weitgehend trockengesaugt. Die anschließende Trocknung führt man zweckmäßig bei möglichst geringem Druck, z.B. < 1 Bar, durch. Die Temperatur bei der Trocknung, die wie oben schon gesagt maximal 60 °C betragen soll, liegt zweckmäßig zwischen 40 und 50 °C.

Die nach dem erfindungsgemäßen Verfahren erhältlichen Erdalkalimetall-Heptandionate der allgemeinen Formel (I) können für all jene Anwendungsbereiche verwendet werden, in welchen solche Metall-Heptandionate üblicherweise verwendet werden. Besonders gut geeignet sind die Verbindungen jedoch zur Anwendung in einem Verfahren zur Abscheidung von Erdalkalimetall enthaltenden Schichten.

Es ist bereits bekannt, Substrate durch Oberflächenbeschichtung, beispielsweise nach dem MOCVD-Verfahren, so zu modifizieren, daß ihre Oberfläche bestimmte funktionale Eigenschaften aufweist. Beispielsweise kann man den elektrischen Strom leitende oder halbleitende Schichten, z.B. Leiterbahnen aufbringen.

Ein Verfahren zur Abscheidung von Erdalkalimetall enthaltenden Schichten unter Anwendung der erfindungsgemäßen Verbindungen der allgemeinen Formel (I) ist ebenfalls Gegenstand der vorliegenden Erfindung.

Das erfindungsgemäße Verfahren zur Abscheidung von Erdalkalimetall enthaltenden Schichten unter Verwendung von Erdalkalimetall-Diketonat-Verbindungen ist dadurch gekennzeichnet, daß man als Erdalkalimetall-Diketonat-Verbindung eine Verbindung der allgemeinen Formel (I)

M(R)₂ (I)

worin M Calcium, Strontium oder Barium und R 2,6-Dimethyl-3,5-heptandionat, 2,2,6-Trimethyl-3,5-heptandionat oder 2,2,6,6-Tetramethyl-3,5-heptandionat bedeutet und in deren ¹H-NMR-Spektrum Wasserprotonen nicht nachweisbar sind, verwendet.

Der Begriff "Erdalkalimetall" umfaßt Calcium, Strontium, Barium oder deren Gemische, wobei die Erdalkalimetalle in der Oxidationsstufe 0, d.h. in metallischer Form in den Schichten enthalten sein können, oder teilweise oder vollständig auch in Form von insbesondere anorganischen Verbindungen vorliegen können, beispielsweise als Oxid, Hydroxid, Carbonat oder Nitrid.

Bevorzugt setzt man in einem solchen Verfahren Verbindungen ein, in welchen R 2,2,6,6-Tetramethyl-3,5-heptandionat bedeutet.

M bedeutet bevorzugt Barium.

Zur Abscheidung einer Barium enthaltenden Schicht verwendet man ganz besonders bevorzugt Barium-bis(2,2,6,6-tetramethyl-3,5-heptandionat).

Die Abscheidung kann der Fachmann aus der kondensierten Phase oder auch aus der Gas- bzw. Dampfphase vornehmen. Für den Fachmann ist dabei selbstverständlich, daß er gewünschtenfalls nicht nur eine bestimmte Verbindung der allgemeinen Formel (I), sondern auch Gemische solcher Verbindungen, in denen M, R oder beide variieren, einsetzen kann.

Zur Abscheidung aus der kondensierten Phase bringt der Fachmann die Verbindung der Formel (I) ohne Lösungsmittel oder vorzugsweise in einem Lösungsmittel gelöst auf dem Substrat auf und zersetzt die Verbindung. Als Lösungsmittel können polare oder unpolare, aprotische organische Lösungsmittel, die gewünschtenfalls koordinierende Eigenschaften aufweisen können, verwendet werden. Geeignet sind beispielsweise aliphatische Kohlenwasserstoffe wie Pentan oder Petrolbenzin, aromatische Kohlenwasserstoffe wie Benzol oder Toluol oder Ether wie Tetrahydrofuran. Weiterhin geeignet sind einbasige oder mehrbasige, insbesondere aliphatische Alkohole, z.B. Methanol, Ethanol, Glykol oder Glykolderivate, ferner insbesondere aliphatische Verbindungen mit einer, zwei oder mehr primären, sekundären oder tertiären Aminogruppen. Das Substrat wird dann in bekannter Weise in die Lösung der Verbindung eingetaucht. Man kann die Ausgangsverbindung oder eine entsprechende Lösung auch beispielsweise auf das Substrat aufsprühen. Auf diese Weise gelingt es immer auch große Flächen sehr schnell zu beschichten.

Dann erfolgt die Zersetzung der auf dem Substrat aufgebrachten Verbindung zur Abscheidung einer Erdalkalimetall enthaltenden Schicht, gewünschtenfalls unter vermindertem Druck. Bevorzugt bewirkt man die Zersetzung thermisch. Dies kann durch Einbringen des mit der Ausgangsverbindung beschichteten Substrat in eine entsprechend beheizte Kammer, durch Heizen des Substrates vor, während und/oder nach dem Aufbringen der Ausgangsverbindung auf den angegebenen Temperaturbereich erfolgen.

Die thermische Zersetzung kann auch strahlungsinduziert bewirkt werden, beispielsweise durch einen Laser, der im UV-Bereich, im Infrarotbereich oder im Bereich des sichtbaren Lichts arbeitet und den Träger aufheizt.

Gewünschtenfalls kann die Zersetzung auch durch Photolyse erfolgen. Die photolytische Zersetzung kann durch einen bei der entsprechenden Wellenlänge betriebenen Laser oder eine UV-Lampe bewirkt werden.

Die Zersetzung kann man auch plasmainduziert durchführen, beispielsweise durch Niederdruckplasmen, z.B. ein Gleichstrom- oder Wechselstromplasma (Niederfrequenz-, Mittelfrequenz, Hochfrequenz-, Mikrowellenplasma) oder Glimmentladungsplasma. Die plasmainduzierte Zersetzung erfolgt in bekannten Plasmareaktoren, beispielsweise Rohr-, Tunnel-, Parallelplatten- oder Koronarentladungsreaktoren. Die Zersetzung im Plasma wird üblicherweise bei erniedrigtem Druck, beispielsweise unterhalb von 10 mbar, z.B. zwischen 10⁻² und 1mbar durchgeführt. Auf diese Weise lassen sich beispielsweise auch Substrate mit verhältnismäßig geringer Thermostabilität, beispielsweise Kunststoffe, beschichten.

Der Fachmann kann durch Zusatz eines Reaktivgases die Form, in welcher das Erdalkalimetall in der Schicht vorliegt, beeinflussen. Dies, sowie die Möglichkeit der gleichzeitigen Abscheidung anderer Metalle oder der sukzessiven Abscheidung weiterer Metalle wird noch erläutert.

Eine andere Ausführungsform des erfindungsgemäßen Verfahrens zur Abscheidung Erdalkalimetall enthaltender Schichten betrifft die Zersetzung der Ausgangsverbindung in der Gas- bzw. Dampfphase. Für diese Ausführungsform sind die erfindungsgemäßen Verbindungen aufgrund der gleichförmigen Verdampfung besonders gut geeignet. Die Abscheidung aus der Gas- bzw. Dampfphase (in der Dampfphase sind neben der gasförmig vorliegenden Ausgangsverbindung auch noch Anteile der kondensiert vorliegenden Ausgangsverbindung in feinster Verteilung enthalten) ermöglicht die Abscheidung besonders gut haftender, gleichmäßiger, dünner Schichten.

Der Druck in der Dampfphase bzw. Gasphase kann mehr oder weniger hoch sein. Man kann beispielsweise bei einem Druck arbeiten, der dem Dampfdruck der verwendeten Ausgansverbindung bei der Arbeitstemperatur entspricht. Der Gesamtdruck kann aber auch höher sein, bis hin zum Normaldruck. Zweckmäßig arbeitet man bei vermindertem Druck, beispielsweise bei 10⁻² bis 10 mbar, vorzugsweise bei 0,1 bis 1 mbar.

Die Zersetzung der Ausgangsverbindung in der Dampfphase oder Gasphase führt man zweckmäßig nach Art eines MOCVD (metal organic chemical vapour deposition-)Verfahren durch.

Die prinzipielle Vorgehensweise zur Beschichtung von Substraten unter Anwendung von MOCVD-Verfahren sowie geeignete Apparaturen dafür sind bekannt. Die EP-A 297 348 (die sich mit anderen Beschichtungen befaßt) gibt dem Fachmann ausführliche Hinweise, wie ein solches Verfahren durchzuführen ist und welche Apparaturen verwendbar sind.

Die Zersetzung aus der Gas- bzw. Dampfphase wird zweckmäßig in einer druckfesten, evakuierbaren Vorrichtung durchgeführt. In diese Vorrichtung wird das zu beschichtende Substrat eingebracht. Bei vermindertem Druck wird eine Atmosphäre erzeugt, welche die Erdalkalimetall enthaltende Ausgangsverbindung der allgemeinen Formel (I) enthält. Neben der gas- bzw. dampfförmigen Ausgangsverbindung kann gewünschtenfalls Inertgas oder Reaktivgas im Gasraum der Vorrichtung vorhanden sein.

In einer Variante wird die Ausgangsverbindung in fester Form zusammen mit dem zu beschichtenden Substrat in die Vorrichtung eingebracht und erst in der Vorrichtung in die Gas- bzw. Dampfphase überführt.

In einer anderen, bevorzugten Variante wird zunächst nur das Substrat in die druckfeste Vorrichtung eingebracht und die bereits gas- bzw. dampfförmig vorliegende Ausgangsverbindung über eine besondere Leitung kontinuierlich oder diskontinuierlich in die Vorrichtung eingebracht. Hier kann ebenfalls ein Trägergas angewendet werden.

Die Überführung der Ausgangsverbindung in die Gas- bzw. Dampfphase kann man durch Erwärmen und gewünschtenfalls durch Zusatz eines Trägergases unterstützen.

Die Zersetzung erfolgt nach bekannten Methoden. Es ist also möglich, die Zersetzung thermisch, durch Einwirkung eines Plasmas und/oder photolytisch zu bewirken.

Die thermische Zersetzung aus der Gas- bzw. Dampfphase führt man üblicherweise so durch, daß die Wände der Vorrichtung kalt gehalten werden und das Substrat auf eine Temperatur erhitzt wird, bei welcher sich die gewünschte Schicht auf dem Substrat abscheidet. Der Fachmann kann durch einfache orientierende Versuche für die jeweils eingesetzte Verbindung die notwendige Mindesttemperatur leicht bestimmen. Üblicherweise liegt die Temperatur, auf welche das Substrat erhitzt wird, oberhalb von etwa 80 °C. Für die Zersetzung von erfindungsgemäßem Ba(TMHD)₂ liegt die Temperatur beispielsweise bei oberhalb von 200 °C.

Die Beheizung der Substrate kann in üblicher Weise erfolgen, beispielsweise durch Widerstandsheizung, Induktionsheizung, elektrische Heizeinrichtungen wie Heizwendeln oder ähnlichem. Die Aufheizung der Substrate kann auch durch Strahlungsenergie induziert werden. Hierfür eignet sich insbesondere Laserstrahlungsenergie. Beispielsweise kann man Laser verwenden, die im Bereich des sichtbaren Lichts, im UV-Bereich oder im IR-Bereich arbeiten. Laser besitzen den Vorteil, daß man sie mehr oder weniger fokussieren kann und daher gezielt bestimmte, begrenzte Bereiche oder Punkte des Substrates erhitzen kann.

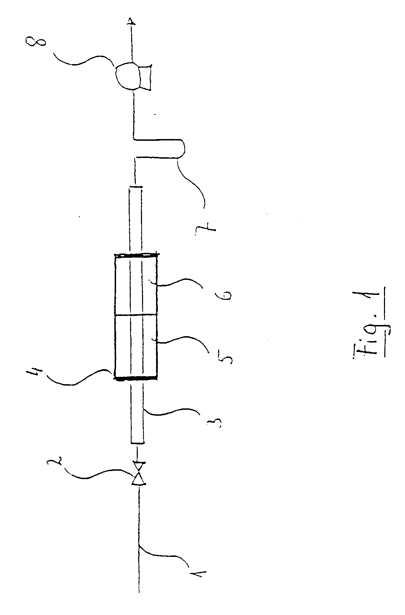

Da das thermische CVD-Verfahren üblicherweise bei Unterdruck durchgeführt wird, ist es für den Fachmann selbstverständlich, druckfeste Apparaturen vorzusehen, wie sie in der Vakuumtechnik verwendet werden. Die Apparaturen weisen zweckmäßigerweise beheizbare Gasleitungen für die metallorganische Verbindung oder das Inertgas, absperrbare Öffnungen für Gasein- und Gasauslaß auf, gegebenenfalls Öffnungen zur Zuführung eines Trägers oder Reaktivgases, Temperaturmeßeinrichtungen, gewünschtenfalls eine Öffnung für die Zuführung der metallorganischen Verbindung, eine Einrichtung für die Aufheizung des Substrats, eine zur Erzeugung des gewünschten Unterdruckes geeignete Pumpe etc. Für den Fall der Durchführung eines durch Strahlungsenergie induzierten CVD-Verfahrens muß auch noch eine Strahlungsquelle vorhanden sein, die Strahlung im Bereich des sichtbaren Lichtes, des Infrarot- oder Ultraviolett-Bereiches abgibt. Besonders geeignet sind entsprechende Laser-Strahlungsenergiequellen. Mittels der Strahlungsenergie kann das Substrat aufgeheizt werden. Eine sehr einfache, zweckmäßige Vorrichtung zur Verfahrensdurchführung ist in Fig. 1 wiedergegeben. Sie umfaßt ein mit einer Inertgaszuleitung 1 über ein absperrbares Ventil 2 verbundenes Glasrohr 3, das konzentrisch in einem röhrenförmig aufgebauten Heizofen 4 angeordnet ist, welcher zwei Heizzonen 5 und 6 aufweist ("Zweizonenröhrenofen"). Die andere Seite des Rohres ist über eine Kühlfalle 7 mit einer Vakuumpumpe 8 verbunden.

In die erste Heizzone, die auf der Seite der Inertgaszuleitung liegt, wird die Ausgangsverbindung eingebracht. In die zweite Heizzone, die auf der Seite der Vakuumpumpe liegt, bringt man das Substrat ein.

Wie schon gesagt, kann die Zersetzung auch photolytisch bewirkt werden. Beispielsweise kann man die Zersetzung plasmainduziert bewirken. Man verwendet wie vorstehend schon beschrieben beispielsweise ein Gleichstrom-, Hochfrequenz-, Mikrowellen- oder Glimmentladungsplasma. Weiterhin kann man die photolytische Zersetzung bewirken, indem man einen mit geeigneter Wellenlänge arbeitenden Laser verwendet.

Ohne daß hier eine Erklärung für die Bildung von Schichten durch Zersetzung der Erdalkalimetallverbindungen gegeben werden soll, wird angenommen, daß Gase bzw. Dämpfe der Verbindung auf das erhitzte Substrat gelangen und dort unter Bildung der Erdalkalimetall enthaltenden Schichten zersetzt werden. Die Dicke der Schichten ist im wesentlichen abhängig von der Zeitdauer, während welcher die Abscheidung durchgeführt wird, vom Partialdruck im Gasraum, und von der Abscheidungstemperatur. Es lassen sich mehr oder weniger dünne Schichten erzeugen, beispielsweise Schichten mit einer Dicke von bis zu 20 Mikrometer, beispielsweise zwischen 100 A und 20 Mikrometer. Je nach gewünschter Schichtdicke kann der Fachmann durch orientierende Versuche die zur Erzeugung einer Schicht bestimmter Dicke notwendige Zeitdauer und Abscheidungstemperatur bestimmen.

Wie schon gesagt, kann die Zersetzung auch photolytisch bewirkt werden, z.B. durch eine UV-Lampe oder durch einen mit geeigneter Wellenlänge arbeitenden Laser.

Der das Substrat umgebende Gasraum enthält die gas- bzw. dampfförmig vorliegende Ausgangsverbindung. Es wurde bereits weiter oben erwähnt, daß weiterhin ein Inertgas oder ein Reaktivgas in der Gasatmosphäre enthalten sein kann. Abhängig von der Art der Durchführung werden Erdalkalimetall enthaltende Schichten abgeschieden, die das Erdalkalimetall in metallischer Form oder aber in Form bestimmter chemischer Verbindungen enthalten.

Zersetzt man die Ausgangsverbindung ohne Zusatz eines Reaktivgases, so scheiden sich Erdalkalimetall enthaltende Schichten ab, die das Erdalkalimetall im wesentlichen in Form von Oxid enthalten.

Die derart beschichteten Substrate können einer Nachbehandlung unterworfen werden.

Zur Erzeugung von Schichten, welche das Erdalkalimetall in metallischer Form enthalten, kann man eine reduzierende Atmosphäre einwirken lassen.

Derartig beschichtete Substrate sind beispielsweise als Getter verwendbar.

Eine Möglichkeit der Nachbehandlung sieht vor, die Schichten einer hydrolysierenden Atmosphäre auszusetzen. Mit Wasser bildet sich zunächst eine Schicht, die ein Erdalkalimetallhydroxid enthält. Erdalkalimetallhydroxide sind bekanntermaßen ebenfalls sehr reaktiv und können beispielsweise mit Kohlendioxid in Erdalkalimetallcarbonat umgewandelt werden. Erdalkalimetallcarbonat wiederum, aber auch Erdalkalihydroxid, kann durch Kalzinieren in Erdalkalimetalloxid überführt werden. Auf diese Weise ist es möglich, mit Erdalkalimetallhydroxid, Erdalkalimetallcarbonat oder Erdalkalimetalloxid beschichtete Substrate herzustellen. Derartig beschichtete Substrate sind beispielsweise verwendbar als optische Schichten.

In einer anderen Ausführungsform führt man die Zersetzung in einer Reaktivgasatmosphäre, insbesondere einer oxidierenden Atmosphäre durch. Anhand der bevorzugten Ausführungsform des erfindungsgemäßen Verfahrens zur Abscheidung Erdalkalimetall enthaltender Schichten, nämlich der Zersetzung in einer oxidierenden Gasatmosphäre, wird diese Variante weiter erläutert. Zweckmäßig führt man die Zersetzung in Anwesenheit einer oxidierenden, beispielsweise Sauerstoff, Stickstoffdioxid oder Ozon enthaltenden Gasatmosphäre durch. Es bilden sich dann Erdalkalimetall enthaltende Schichten, welche das Erdalkalimetall in Form von Erdalkalimetalloxid enthalten. Alternativ kann man auch in einer hydrolysierenden oder carbonisierenden Atmosphäre arbeiten, beispielsweise in Anwesenheit von Wasser und Kohlendioxid. Das intermediär entstehende Erdalkalimetallcarbonat oder Hydroxid wird dann zu Metalloxid kalziniert. Einfacher ist es jedoch, die Zersetzung in Anwesenheit einer insbesondere Sauerstoff enthaltenden Atmosphäre durchzuführen.

Im Anschluß an die Abscheidung in der oxidierenden Reaktivgasatmosphäre kann man gewünschtenfalls die abgeschiedenen Schichten noch einer Nachbehandlung, insbesondere einer oxidierenden Nachbehandlung, unterwerfen.

Das erfindungsgemäße Verfahren eignet sich auch zur Abscheidung von Schichten, welche neben dem Erdalkalimetall ein oder mehrere andere Metalle enthalten. Diese Ausführungsform des erfindungsgemäßen Abscheidungsverfahrens ist dadurch gekennzeichnet, daß man zur Abscheidung von Erdalkalimetall enthaltenden Schichten, welche weiterhin ein oder mehrere andere Metalle enthalten, eine oder mehrere Verbindungen anderer Metalle und eine Verbindung der allgemeinen Formel (I) gleichzeitig oder nacheinander zersetzt.

Sofern man die Abscheidung aus kondensierter Phase vornimmt, verwendet man Gemische, die neben einer Ausgangsverbindung der allgmeinen Formel (I) beliebige bekannte zersetzbare Verbindungen enthalten. Besonders bevorzugt zersetzt man unter Bildung von Schichten, die das Erdalkalimetall und das oder die weiteren Metalle in Form der Metalloxide enthalten. Hierzu kann man, wie oben beschrieben, in Reaktivgasatmosphäre arbeiten und gewünschtenfalls die beschichteten Substrate einer oxidierenden Nachbehandlung unterwerfen. Beispielsweise kann man zersetzbare Titanverbindungen etwa Titanalkoholate oder Titan-β-Diketonate einsetzen und Erdalkalimetalltitanate, insbesondere Bariumtitanat, enthaltende Schichten abscheiden. Natürlich kann man auch modifizierte Erdalkalimetalltitanate herstellen. Beispielsweise kann man einen Teil des Bariums durch Blei, Calcium oder Strontium und einen Teil des Titans durch Zinn, Zirkonium oder Hafnium ersetzen. Man geht dann zweckmäßig von β-Diketonaten dieser Verbindungen aus. Bei der Abscheidung aus der kondensierten Phase kann man nach Art des bekannten Sol-Gel-Verfahrens arbeiten.

Die hierbei hergestellten Titanate besitzen dielektrische Eigenschaften und die mit diesen Dielektrika beschichteten Substrate können in der Elektro- und Sensortechnik verwendet werden.

Weiterhin eignen sich die erfindungsgemäßen Verbindungen der Formel (I) auch zur Anwendung in einem erfindungsgemäßen, aus der kondensierten Phase durchgeführten Abscheidungsverfahren zur Herstellung von Erdalkalimetall enthaltenden oxidischen Gemischen, die in ihrer chemichen Zusammensetzung bekannten hochtemperatursupraleitenden Keramiken, beispielsweise des Typs Seltenerdmetall-Erdalkalimetall-Kupfer-Oxid entsprechen. Beispielsweise kann man β-Diketonate von Yttrium und Kupfer und eine Bariumverbindung der allgemeinen Formel (I) mischen, zersetzen, gewünschtenfalls mit Sauerstoff nachbehandeln und gewünschtenfalls tempern. Man erhält dann ein Yttrium-Barium-Kupfer-Oxid.

Gewünschtenfalls kann man auch modifizierte Gemische herstellen, indem man einen Teil der Yttrium-, Barium- und/oder Kupferverbindungen durch zersetzbare Verbindungen anderer Metalle ersetzt, beispielsweise durch erfindungsgemäße oder bekannte Calciumverbindungen, erfindungsgemäße oder bekannte Strontiumverbindungen, durch Lanthanverbindungen, Wismutverbindungen, Thalliumverbindungen oder durch andere Seltenerdmetallverbindungen. Das gewünschte Molverhältnis der Metalle kann beim Vermischen auf einfache Weise eingestellt werden.

Besonders gut eignen sich die erfindungsgemäßen Verbindungen der allgemeinen Formel (I) zur Abscheidung in der Gas- bzw. Dampfphase, weil sie eine sehr gleichförmige Verdampfungsrate besitzen.

Ganz besonders gut eignen sich die erfindungsgemäßen Verbindungen der Formel (I) zur Herstellung von Schichten, die in ihrer chemischen Zusammensetzung oxidkeramischen Hochtemperatursupraleitern entsprechen, insbesondere solchen des Seltenerdmetall-Erdalkalimetall-Kupfer-Oxid-Typs. Aus der Gas- bzw. Dampfphase lassen sich besonders dünne, guthaftende, gleichförmige Schichten erzeugen.

Als Träger verwendet man die bei CVD-Verfahren üblicherweise verwendeten Träger. Zur Abscheidung supraleitender Schichten kann man als Träger beispielsweise Strontiumtitanat, Aluminiumoxid, Magnesiumoxid oder andere bekanntermaßen verwendbare Materialien verwenden. Diese Substrate können beispielsweise in Form von Fasern, beispielsweise als Kohlenstoffasern, in Form von Bändern, Folien, Rohren oder in Form von Platten ausgebildet sein.

Zur Abscheidung von oxidkeramischen Hochtemperatursupraleitern, beispielsweise solchen des Seltenerdmetall-Erdalkalimetall-Kupfer-Oxid-Typs wie Y₁Ba₂Cu₃Oₓ, worin x etwa 6,8 bis 6,95 bedeutet, scheidet man zweckmäßig die verschiedenen Metallschichten nacheinander ab. Die Abscheidung des Erdalkalimetalls erfolgt hierbei nach dem erfindungsgemäßen Verfahren.

Vorteilhafterweise scheidet man zunächst eine Kupfer enthaltende Schicht ab, indem man eine bekannte verdampfbare Kupfer-Verbindung, beispielsweise Kupferacetylacetonat, Kupfer-TMHD oder Cyclopentadienyl-Kupfer-Trialkylphosphinkomplexe, bei vermindertem Druck und einer Temperatur zwischen Raumtemperatur und etwa 300 °C oder höher verdampft und aus oxidierender Gasatmosphäre auf dem Träger abscheidet.

Sehr gut geeignet zur Abscheidung Kupfer enthaltender Schichten sind auch die aus der deutschen Patentanmeldung DE-A 41 22 449 bekannten Kupferverbindungen der allgemeinen Formel RO-Cu-L (II). R bedeutet insbesondere verzweigtes Alkyl mit 3 bis 6 C-Atomen, L steht für t-Butylisonitril, Kohlenmonoxid sowie bestimmte dreiwertige Phosphorverbindungen. Verbindungen dieser Art lassen sich leicht herstellen, indem man das betreffende Lithium-Alkoholat zunächst mit Kupfer(I)chlorid zum Zwischenprodukt RO-Cu umsetzt und dieses mit dem Liganden L zu der gewünschten Verbindung der allgemeinen Formel (II) weiter reagieren läßt. Sehr gut geeignet sind insbesondere die aus jener Patentanmeldung bekannten Verbindungen t-Butoxy-Kupfer-t-butyldifluorphosphan, t-Butoxy-Kupfer-phenyldifluorphosphan, t-Butoxy-Kupfer-diethylaminodifluorphosphan, 2-Trifluormethylpropan-2-oxy-Kupfer-t-butylisonitril, 2-Trifluormethylpropan-2-oxy-Kupfer-trimethylphosphan, Perfluor-t-Butoxy-Kupfer-t-butylisonitril und Perfluor-t-Butoxy-Kupfer-trimethylphosphan.

Sehr gut geeignet sind auch die in der deutschen Patentanmeldung DE-A 41 24 686 als brauchbar beschriebenen Verbindungen der allgemeinen Formel R-Cu-L (III). Hier steht R für Cyclopentadienyl oder durch 1 bis 5 Substituenten, insbesondere Alkyl mit 1 bis 5 C-Atomen substituiertes Cyclopentadienyl. L steht für (C₁-C₆-Alkyl)isonitril oder Arylisonitril. Verbindungen dieser Art lassen sich herstellen, indem man wasserfreies Kupfer(I)chlorid mit t-BuOLi zu t-BuO-Cu umsetzt, dies mit einem Isonitril, beispielsweise t-BuNC zu t-BuO-Cu-t-BuNC umsetzt. Diese Zwischenverbindung wird dann mittels Cyclopentadien bzw. einem substituierten Cyclopentadien in die gewünschte Verbindung der allgemeinen Formel (III) überführt. Sehr gut geeignet ist beispielsweise C₅H₅-Cu-t-BuNC. Nach Abscheidung der Kupfer enthaltenden Schicht scheidet man zweckmäßig die Seltenerdmetall enthaltende Schicht ab. Beispielsweise verdampft man Verbindungen des Yttriums, insbesondere β-Diketonate wie Yttrium-tris(2,2,6,6-tetramethyl-3,5-heptandionat). Nach der Zersetzung dieser Verbindungen verdampft und zersetzt man zur Abscheidung der Erdalkalimetall enthaltenden Schicht eine erfindungsgemäße Verbindung der allgemeinen Formel (I), beispielsweise Barium-bis-2,2,6,6-tetramethyl-3,5-heptandion. Die Zersetzungstemperatur beträgt etwa 500 °C bis 700 °C.

Gewünschtenfalls kann die vorstehend beschriebene Reihenfolge der Schichtabscheidung auch verändert werden. Zur Überführung der abgeschiedenen Oxidschicht in einem Hochtemperatursupraleiter wird die Beschichtung dann noch getempert.

In völlig analoger Weise können modifizierte oxidkeramische Schichten hergestellt werden, indem man das gesamte Yttrium oder einen Teil davon durch Strontium, Lanthan, Thallium, Wismut oder andere Metalle ersetzt. Zusätzlich oder stattdessen kann man auch das gesamte Barium bzw. einen Teil davon durch andere Erdalkalimetalle ersetzen. Zweckmäßig verwendet man Metall-β-Diketonate. Zur Abscheidung Erdalkalimetall enthaltender Schichten, die zusätzlich zu Barium oder anstelle von Barium andere Erdalkalimetalle enthalten, verwendet man die erfindungsgemäßen Verbindungen der allgemeinen Formel (I), in welchen M Calcium bzw. Strontium bedeutet.

Auf diese Weise kann man neben gegebenenfalls modifizierten Supraleitern des Seltenerdmetall-Erdalkalimetall-Kuprat-Typs auch Supraleiter auf Thalliumoxid- bzw. Wismutoxid-Basis herstellen.

Abgesehen von der vorstehend beschriebenen Methode, Hochtemperatursupraleiter durch sukzessive Abscheidung von Schichten zu erzeugen, kann man natürlich auch von Gemischen ausgehen und dann homogene Schichten abscheiden.

Ferner kann man außer der vorstehend beschriebenen Abscheidung aus der Gas- bzw. Dampfphase auch, wie weiter oben beschrieben, aus der kondensierten Phase abscheiden.

Die Dicke der abgeschiedenen supraleitenden Schicht beträgt zweckmäßig etwa 5 bis 20 Mikrometer.

Die folgenden Beispiele sollen die Erfindung weiter erläutern, ohne sie in ihrem Umfang einzuschränken.

### Beispiel 1

### Herstellung von Barium-bis(2,2,6,6-tetramethyl-3,5-heptandionat) (erfindungsgemäß)

In einem 250 ml-Rundkolben wurden 22,08 g Bariumhydroxid-Octahydrat (0,07 mol) in 120 ml destilliertem Wasser aufgeschlämmt. Zu der weißen Suspension wurden 25,8 g (0,14 mol) 2,2,6,6-Tetramethyl-3,5-heptandion zugetropft. Es bildete sich ein weißer Feststoff. Die Reaktionsmischung wurde noch 10 Stunden lang bei 50 °C Ölbadtemperatur weitergerührt. Das weiße Produkt wurde dann nach dem Abkühlen auf Raumtemperatur über eine Nutsche abfiltriert. Zur Beschleunigung der Filtration wurde ein Wasserstrahlvakuum angelegt. Der abfiltrierte Feststoff wurde dann mit destilliertem Wasser nachgewaschen und im Wasserstrahlvakuum weitgehend trockengesaugt. Die Feuchtausbeute betrug 40,5 g. Das vorgetrocknete Produkt wurde dann im Ölpumpenvakuum bei etwa 10⁻² bis 10⁻³ Torr unter Aufwärmen auf 50 bis 55 °C (Ölbadheizung) 4 Stunden lang getrocknet. Die Ausbeute betrug 33,5 g (95,7 % d.Th.).

| Elementaranalyse: | |
|---|---|
| | Ba |
| ber. | 27,25 % |
| gef. | 27,1 % |

Im ¹H-NMR-Spektrum einer Lösung des erfindungsgemäßen Ba(TMHD)₂ in D₆-DMSO war auch bei einem Signal-Rausch-Verhältnis von >600 kein Signal für H₂O-Protonen identifizierbar.

Röntgenspektrum: 2 Theta = 23,99°; 16,7°; 6,3°; 7,0°;
(ausgewählte Peaks) 7,4 °; 5,6°.

### Bestimmung der Gewichtsabnahme bei 200 °C im Vakuum:

Verwendete Apparatur: NETZSCH Simultane Thermoanalysenapparatur STA 409

### Durchführung:

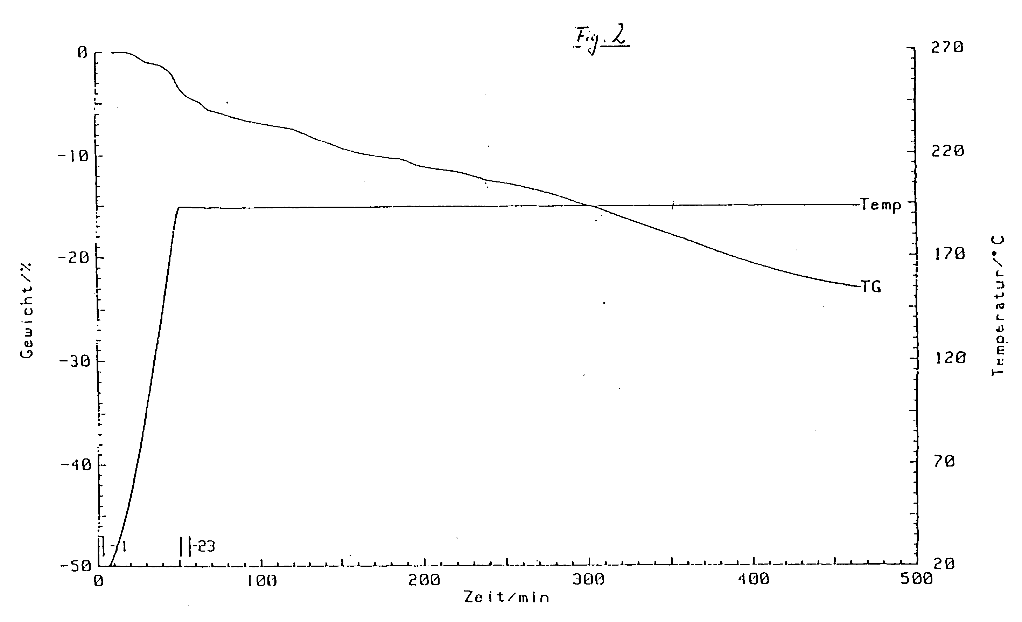

Zur Bestimmung der Gewichtsabnahme wurde eine Probe von 81,00 mg des in Beispiel 1 hergestellten erfindungsgemäßen Ba(TMHD)₂ in einen Aluminiumoxidtiegel gegeben und in die Thermoanalysenapparatur eingebracht. Als Referenz diente Aluminiumoxid (56,00 mg). Die Atmosphäre in der Apparatur bestand aus Stickstoff, der Druck betrug 10 Torr. Nach Einbringen der Probe wurde die Temperatur in der Analysenapparatur auf 200 °C gebracht. Diese Temperatur wurde nach einer halben Stunde erreicht. Die Veränderung des Gewichts der Probe wurde, beginnend mit dem Einbringen der Probe in die Thermoanalysenapparatur, über einen Zeitraum von etwa 460 Minuten mittels eines Schreibers aufgezeichnet. Der zugehörige Graph ist in Fig. 2 wiedergegeben. In der Abszisse ist in der Einheit "min" die Versuchsdauer vom Zeitpunkt 0 (Einbringen der Probe in die Analysenapparatur) bis zum Abbruch des Versuchs nach etwa 470 Minuten aufgetragen. In der linken Ordinate ist die Gewichtsabnahme während des Versuches in Form negativer Prozentzahlen, jeweils bezogen auf das ursprünglich eingesetzte Gesamtgewicht der Probe, aufgetragen. Die rechte Ordinate gibt die Temperatur an. Das ursprüngliche Gewicht der in die Analysenapparatur eingebrachten Probe wurde willkürlich gleich 0 gesetzt. Eine Probe mit einer Gewichtsabnahme von "-10" % entspricht dann einer Probe, die noch 90 Gew.-% ihres ursprünglichen Gewichtes aufweist. Die zugehörige Kurve, die die Gewichtsabnahme über die Versuchsdauer wiedergibt, ist in der Fig. 2 mit "TG" bezeichnet. Die Kurve, die den Temperaturverlauf während des Versuches wiedergibt, ist in der Fig. 2 mit "Temp" bezeichnet.

In der Tabelle 1 sind aus der Fig. 2 entnehmbare Werte für die Gewichtsabnahme der Probe und die korrespondierenden, daraus berechneten Restgewichte der Probe zu bestimmten Meßzeitpunkten t₀, t₁ etc. zusammengestellt.

**Tabelle 1**

| | Meßzeitpunkt (min) | Gew.-Abnahme der Probe (Gew.-%) | ber. Restgewicht der Probe (Gew.-%) |
|---|---|---|---|
| t₀* | 0 | 0 | 100 |
| t₁** | 50 | - 3,5 | 96,5 |
| t₂ | 110 | - 7 | 93 |
| t₃ | 170 | -10 | 90 |
| t₄ | 230 | -12,5 | 87,5 |
| t₅ | 290 | -15 | 85 |
| t₆ | 350 | -18 | 82 |
| t₇ | 410 | -21,5 | 78,5 |

| | | | |
|---|---|---|---|
| * t₀ = Zeitpunkt des Versuchsbeginns | | | |
| ** t₁ = Erreichen der vorbestimmten Temperatur in der Analysenapparatur (200 °C) | | | |

Aus Tabelle 1 ist zu entnehmen, daß während einer Versuchsdauer von 410 Minuten die Gewichtsabnahme pro Zeiteinheit in einem nur gering schwankenden Bereich liegt. So beträgt die durchschnittliche Gewichtsabnahme zwischen dem Zeitpunkt t₁ (Erreichen der vorbestimmten Temperatur in der Analysenapparatur) bis zum Zeitpunkt t₇ etwa -18 Gew.-%. Die durchschnittliche Gewichtsabnahme zwischen zwei aufeinanderfolgenden Meßzeitpunkten beträgt dann bezogen auf das Gewicht der Probe zum Zeitpunkt t₀ (Versuchsbeginn), etwa -3 Gew.-%. Ein Blick in die Tabelle 1 zeigt, daß die tatsächliche Gewichtsabnahme zwischen zwei aufeinanderfolgenden Meßzeitpunkten zwischen -2,5 Gew.-% (Differenz der Ergebnisse zum Meßzeitpunkt t₃ und t₄ bzw. t₄ und t₅) und -3,5 Gew.-% (Gewichtsabnahme zwischen den Meßzeitpunkten t₁ und t₂ bzw. t₆ und t₇) schwankt. Eine solch geringe Schwankungsbreite ist sehr vorteilhaft bei der Anwendung in CVD-Verfahren.

### Bestimmung der Verdampfungsrate:

### Verwendete Apparatur:

Im wesentlichen umfaßte die Vorrichtung ein evakuierbares Gefäß, das mit einer Vakuumpumpe verbunden war sowie mit einer Gaszuleitung, durch die ein Trägergas in das Gefäß eingeleitet werden konnte. Über eine Gasvorheizung konnte das Trägergas auf eine vorgegebene Temperatur erhitzt werden. Das Gefäß war weiterthin gasdicht mit einer Mikrowaage verbunden, deren Meßsignale über eine Elektronik und einen Rechner auf einen Schreiber einwirkten. Die Temperatur der Probe konnte über die Gasvorheizung und entsprechende Thermoelemente kontrolliert und geregelt werden.

### Durchführung:

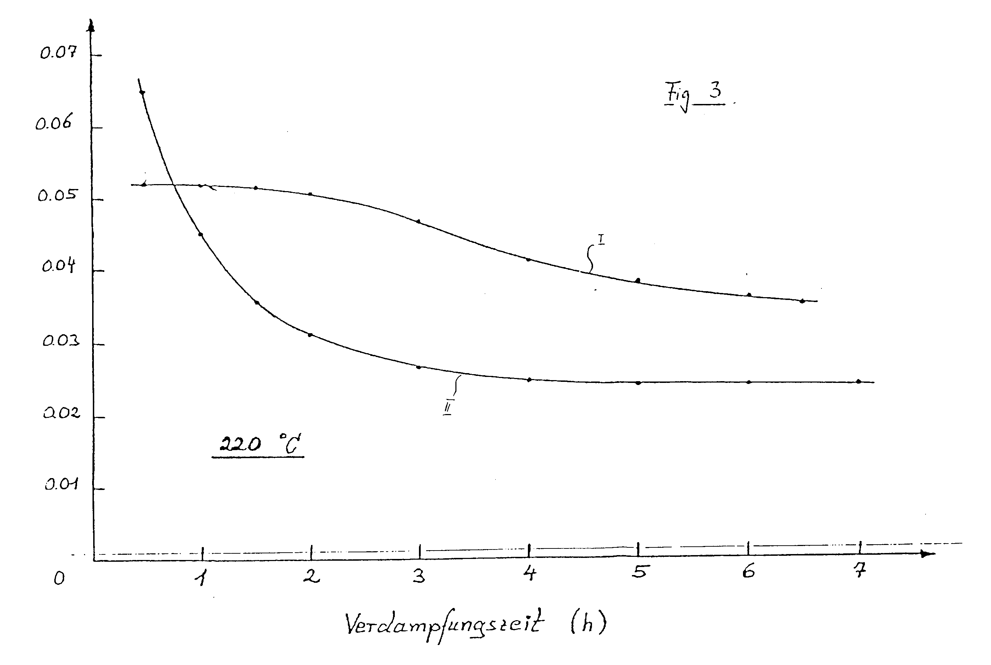

Die Meßprobe wurde in einem kleinen Tiegel in das evakuierbare Gefäß eingebracht, und zwar derart, daß der Tiegel freischwebend an einem Faden aufgehängt war, der mit der Mikrowaage verbunden war, so daß Gewichtsabnahmen auf die Mikrowaage einwirkten. Die Gasvorheizung wurde derart reguliert, daß die Meßprobe eine Temperatur von 220 °C aufwies. Mit einer Ölpumpe wurde dann ein Vakuum (10 mbar) erzeugt. Die von der Mikrowaage registrierten Gewichtsänderungen in der Meßprobe wurden vom Rechner in eine Verdampfungsrate in den Einheiten mg/min umgewandelt und vom Schreiber aufgezeichnet. In Fig. 3, Graph I ist der Verlauf der Verdampfungsrate einer Probe des in Beispiel 1 hergestellten Ba(TMHD)₂ bei 220 °C während einer Zeitdauer von 6 Stunden wiedergegeben. In Tabelle 2 sind aus der Fig. 3 entnehmbare Zahlenwerte für die Verdampfungsrate nach etwa 30 Minuten sowie nach 1, 2, 3, 4, 5 und 6 Stunden aufgelistet.

**Tabelle 2**

| | Meßzeitpunkt (h) | Verdampfungsrate (mg/min) |
|---|---|---|
| t₀* | 0 | -- |
| t₁ | 0,5 | 0,052 |
| t₂ | 1 | 0,0518 |
| t₃ | 2 | 0,050 |
| t₄ | 3 | 0,047 |
| t₅ | 4 | 0,041 |
| t₆ | 5 | 0,038 |
| t₇ | 6 | 0,036 |

| | | |
|---|---|---|
| * t₀ = Beginn der Messung | | |

Wie der Tabelle 2 zu entnehmen ist, sinkt die Verdampfungsrate zwischen dem Meßzeitpunkt t₁ und t₇ von 0,052 mg/min auf 0,036 mg/min. Das bedeutet, daß 5½ Stunden nach Beginn der Messung die Verdampfungsrate noch immer 69,2 % des ursprünglichen Wertes beträgt. Außerdem nimmt die Verdampfungsrate gleichförmig ab. Aufgrund dieser nur geringfügigen, zudem gleichförmigen Abnahme der Verdampfungsrate ist das erfindungsgemäße Ba(TMHD)₂ sehr gut für die Anwendung bei der CVD-Synthese geeignet.

### Beispiel 2 (Vergleichsbeispiel):

### Herstellung von Ba(TMHD)₂ aus wasserfreien Lösungsmitteln

In einem 250 ml-Rundkolben wurden 37,8 g Bariumhydroxid-Octahydrat (0,12 mol) vorgelegt. Über einen Tropftrichter wurden 50 ml 2,2,6,6-Tetramethyl-3,5-heptandion (0,24 mol) zugetropft. Dann wurden 70 ml Xylol zugegeben. Das Reaktionsgemisch wurde dann mittels eines Ölbades auf 50 °C erhitzt und 10 Stunden lang bei dieser Temperatur gerührt. Anschließend wurde die Ölbadtemperatur auf 60 °C gebracht und flüchtige Bestandteile am Hochvakuum abgedampft.
Trockenausbeute: 57,5 g (95 % der Theorie)

Gemäß der Elementaranalyse war 1 % mehr Barium als berechnet in der Probe enthalten. Daher wurde das hergestellte Ba(TMHD)₂ mit 100 ml Hexan versetzt und weitere 8 ml TMHD (7,16 g, entsprechend 0,0384 mol) zugegeben. Dann wurde das Reaktionsgemisch bei einer Temperatur von 50 °C noch einmal 8 Stunden gerührt und anschließend am Hochvakuum, wie oben beschrieben, getrocknet.

| Elementaranalyse: | |
|---|---|
| | Ba [%] |
| ber. | 27,25 |
| gef. | 27,4 |

Im ¹H-NMR-Spektrum einer Lösung des Produkts in D₆-DMSO ist deutlich ein Signal von Protonen des Wassers zu identifizieren.

### Bestimmung der Verdampfungsrate

### Verwendete Apparatur:

Es wurde die in Beispiel 1 beschriebene Apparatur zur Bestimmung der Verdampfungsrate verwendet.

Die Durchführung erfolgte analog zu der in Beispiel 1 beschriebenen Durchführung. Der Verlauf der Verdampfungsrate ist in Fig. 3, Graph II wiedergegeben. Die daraus berechneten Meßergebnisse sind in Tabelle 3 zusammengestellt.

**Tabelle 3**

| (Vergleichsbeispiel 2) | | |
|---|---|---|
| | Meßzeitpunkt (h) | Verdampfungsrate (mg/min) |
| t₀* | 0,5 | 0,065 |
| t₁ | 1 | 0,045 |
| t₂ | 2 | 0,031 |
| t₃ | 3 | 0,027 |
| t₄ | 4 | 0,0245 |
| t₅ | 5 | 0,024 |
| t₆ | 6 | 0,024 |

| | | |
|---|---|---|
| * t₀ = Beginn der Messung | | |

Aus der Tabelle ist zu entnehmen, daß die Verdampfungsrate von ursprünglich 0,065 mg/min nach 5½ Stunden auf einen Wert von nur noch 0,024 mg/min abgefallen ist. Dies sind nur noch 37 % des ursprünglichen Wertes. Außerdem ist der Abfall der Verdampfungsrate anfangs sehr groß. Ein solch starker, ungleichförmiger Abfall ist sehr unvorteilhaft bei der Anwendung.

### Beispiel 3

### Herstellung von Calcium-bis(2,2,6,6-tetramethyl-3,5-heptandionat) (erfindungsgemäß)

In einem 250 ml-Rundkolben wurden 3,7 g Calciumhydroxid (0,05 mol) in etwa 100 ml destilliertem Wasser aufgeschlämmt. Zu der weißen Suspension wurden unter Rühren 18,4 g TMHD (0,1 mol) zugetropft. Es bildete sich ein weißer Feststoff. Die Reaktionsmischung wurde dann mittels eines Ölbades auf eine Temperatur von 50 °C gebracht und 10 Stunden lang bei dieser Temperatur gerührt. Nach dem Abkühlen auf Raumtemperatur wurde das weiße Produkt über eine Nutsche abfiltriert und mit destilliertem Wasser nachgewaschen. Die Feuchtausbeute nach dem weitgehenden Trockensaugen im Wasserstrahlvakuum betrug 25,0 g. Anschließend wurde der Feststoff mittels eines auf 50 °C temperierten Ölbades erwärmt und im Hochvakuum (Ölpumpe) bei etwa 10⁻² bis 10⁻³ Torr getrocknet.
Trockenausbeute: 18,8 g (94 % der Theorie)

### Schmelzverhalten:

Unmittelbar nach der Herstellung: bei 255 °C Bildung einer trüben Schmelze.

Nach 30 Tagen: Sinterung bei 245 °C, Bildung einer trüben Schmelze bei 250 °C.

| Elementaranalyse: | |
|---|---|
| | Ca |
| ber. | 9,86 |
| gef. | 10,2 |

### Bestimmung der Gewichtsabnahme bei 200 °C im Vakuum:

Es wurde wiederum die auch in Beispiel 1 verwendete Apparatur der Firma Netzsch eingesetzt. Die Gewichtsabnahme wurde a) in einer Luftatmosphäre und b) in einer Stickstoffatmosphäre durchgeführt.

### a) Durchführung in Luftatmosphäre:

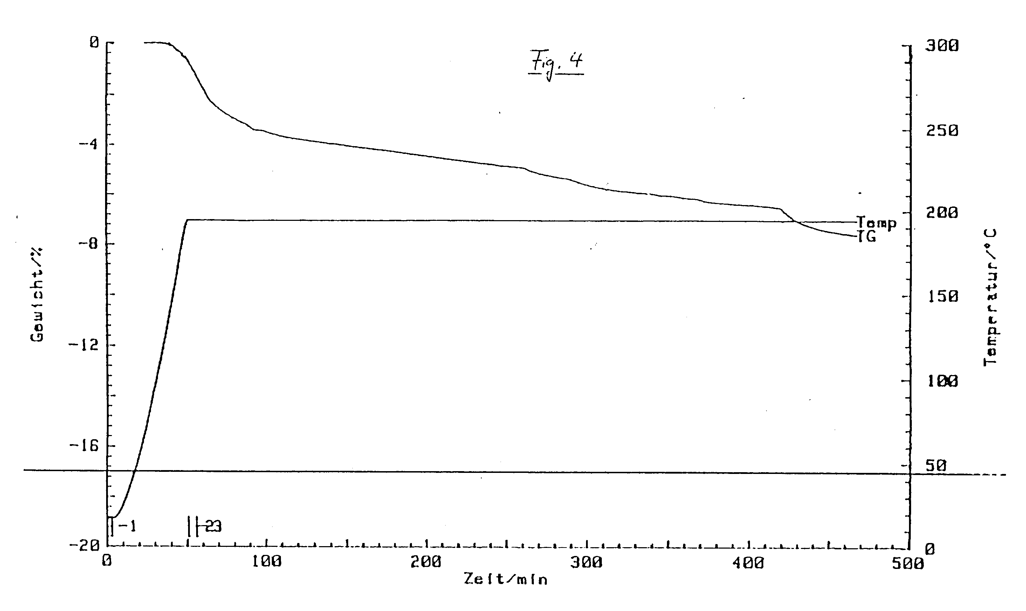

Zur Bestimmung der Gewichtsabnahme wurde eine Probe von 93,00 mg des in Beispiel 3 hergestellten erfindungsgemäßen Ca(TMHD)₂ in einem Aluminiumoxidtiegel gegeben und in die Thermoanalysenapparatur eingebracht. Als Referenz diente Aluminiumoxid (90,00 mg). Die Atmosphäre in der Apparatur bestand wie gesagt aus Luft, der Druck betrug etwa 10 Torr. Nach Einbringen der Probe wurde die Temperatur in der Analysenapparatur binnen einer halben Stunde auf 200 °C gebracht. Die Veränderung des Gewichts der Probe wurde wie schon in Beispiel 1 beschrieben über einen Zeitraum von 460 Minuten mittels eines Schreibers aufgezeichnet. Der zugehörige Graph ist in Fig. 4 wiedergegeben. Die Kurve, die die Gewichtsabnahme über die Versuchsdauer wiedergibt, ist wiederum mit "TG" und die Kurve des Temperaturverlaufes mit "Temp" bezeichnet.

In der Tabelle 4 sind aus der Fig. 4 entnehmbare Werte für die Gewichtsabnahme der Probe und die korrespondierenden, daraus berechneten Restgewichte der Probe zu bestimmten Meßzeitpunkten t₀, t₁ etc. zusammengestellt.

**Tabelle 4**

| | Meßzeitpunkt (min) | Gew.-Abnahme der Probe (Gew.-%) | ber. Restgewicht der Probe (Gew.-%) |
|---|---|---|---|
| t₀* | 0 | 0 | 100 |
| t₁** | 50 | - 0,77 | 99,23 |
| tₐ | 80 | - 3 | 97 |
| t₂ | 110 | - 3,7 | 96,3 |
| t₃ | 170 | - 4,3 | 95,7 |
| t₄ | 230 | - 4,8 | 95,2 |
| t₅ | 290 | - 5,5 | 94,5 |
| t₆ | 350 | - 6,1 | 93,9 |
| t₇ | 410 | - 6,5 | 93,5 |

| | | | |
|---|---|---|---|
| * t₀ = Zeitpunkt des Versuchsbeginns | | | |
| ** t₁ = Erreichen der vorbestimmten Temperatur in der Analysenapparatur (200 °C) | | | |

Aus Tabelle 4 ist zu entnehmen, daß bereits 30 Minuten (tₐ) nach Erreichen der vorgegebenen Temperatur von 200 °C in den darauffolgenden 330 Minuten die Gewichtsabnahme pro Zeiteinheit sehr gleichmäßig ist. Der absolute Betrag der Gewichtsabnahme pro Stunde liegt im Bereich von (0,56 ± 0,16) Gew.-%. Aufgrund dieser geringen Schwankungsbreite kann das erfindungsgemäße Ca(TMHD)₂ sehr vorteilhaft in CVD-Verfahren eingesetzt werden.

### b) Durchführung in Stickstoffatmosphäre:

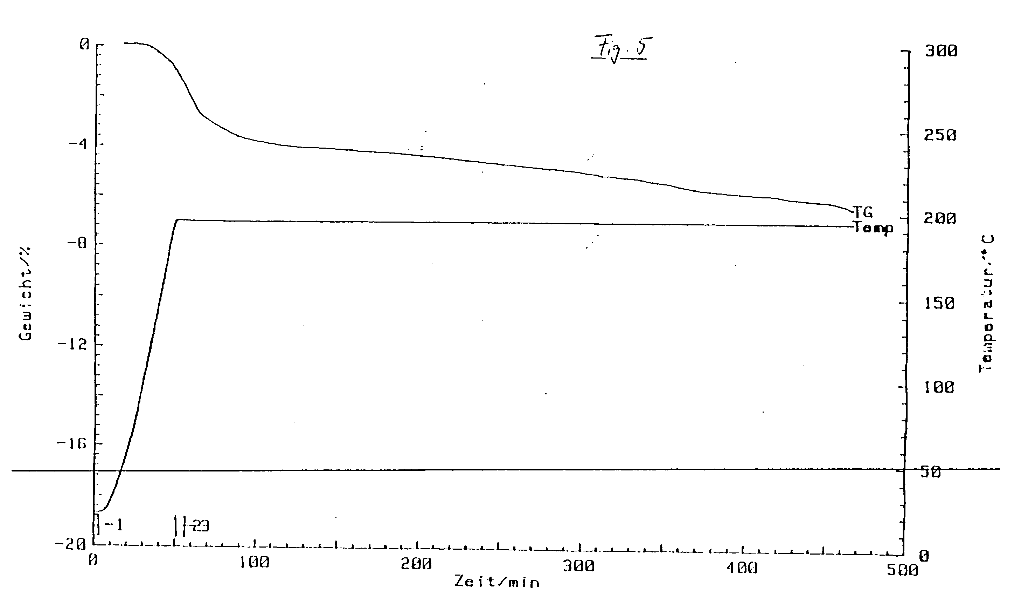

Diesmal wurde eine Probe von 83,00 mg des in Beispiel 3 hergestellten erfindungsgemäßen Ca(TMHD)₂ in einen Aluminiumoxidtiegel gegeben und in die Thermoanalysenapparatur eingebracht. Als Referenz diente Aluminiumoxid (89,00 mg). Die Atmosphäre in der Apparatur bestand aus Stickstoff, der Druck betrug etwa 10 Torr. Nach Einbringen der Probe wurde die Temperatur in der Analysenapparatur binnen einer halben Stunde auf etwa 200 °C gebracht. Die Veränderung des Gewichts der Probe wurde, beginnend mit dem Einbringen der Probe in die Thermoanalysenapparatur, über einen Zeitraum von etwa 460 Minuten mittels eines Schreibers aufgezeichnet. Der zugehörige Graph ist in Fig. 5 wiedergegeben. Die Kurve, die die Gewichtsabnahme über die Versuchsdauer wiedergibt, ist in der Fig. 5 wiederum mit "TG", und die den Temperaturverlauf wiedergebende Kurve mit "Temp" bezeichnet.

In der Tabelle 5 sind aus der Fig. 5 entnehmbare Werte für die Gewichtsabnahme der Probe und die korrespondierenden, daraus berechneten Restgewichte der Probe zu bestimmten Meßzeitpunkten t₀, t₁ etc. zusammengestellt.

**Tabelle 5**

| | Meßzeitpunkt (min) | Gew.-Abnahme der Probe (Gew.-%) | ber. Restgewicht der Probe (Gew.-%) |
|---|---|---|---|
| t₀* | 0 | 0 | 100 |
| t₁** | 50 | - 1,1 | 98,9 |
| tₐ | 80 | - 3,3 | 96,7 |
| t₂ | 110 | - 3,9 | 96,1 |
| t₃ | 170 | - 4,2 | 95,8 |
| t₄ | 230 | - 4,45 | 95,55 |
| t₅ | 290 | - 4,9 | 95,1 |
| t₆ | 350 | - 5,3 | 94,7 |
| t₇ | 410 | - 5,8 | 94,2 |

| | | | |
|---|---|---|---|
| * = Zeitpunkt des Versuchsbeginns | | | |
| ** = Erreichen der vorgestimmten Temperatur in der Analysenapparatur (200 °C) | | | |

Aus Tabelle 5 ist zu entnehmen, daß auch in Stickstoffatmosphäre die Gewichtsabnahme pro Zeiteinheit über einen Verlauf von mehreren Stunden nur gering schwankt. Zwar ist in den ersten 30 Minuten nach Erreichen der vorgegebenen Temperatur von 200 °C noch eine überdurchschnittlich starke Gewichtsabnahme der Probe zu beobachten. Etwa 1 Stunde nach Erreichen der vorgegebenen Temperatur verdampft die Probe sehr gleichförmig. Der absolute Wert der Gewichtsabnahme liegt in einem Bereich von etwa (0,38 ± 0,17) Gew.-% pro Stunde. Die Anwendung im CVD-Verfahren ist somit auch in Stickstoffatmosphäre sehr vorteilhaft.

### Beispiel 4

### Herstellung von Strontium-bis(2,2,6,6-tetramethyl-3,5-heptandionat)

In einem 250 ml-Rundkolben wurden 11,69 g Strontiumhydroxid-Octahydrat (0,044 mol) in etwa 100 ml destilliertem Wasser aufgeschlämmt. Unter Rühren wurden dann 16,22 g TMHD (0,088 mol) zugetropft. Es fiel ein Feststoff aus. Dieser Feststoff wurde mit einem Spatel zerkleinert und die Reaktionsmischung dann noch 10 Stunden lang unter Erhitzen mit einem auf 50 °C temperierten Ölbad weitergerührt. Der Feststoff wurde danach mit einer Nutsche abfiltriert, mit destilliertem Wasser gewaschen und mit einer Wasserstrahlpumpe vorgetrocknet. Die Feuchtausbeute betrug 24,2 g. Anschließend wurde der Feststoff unter Erwärmen mit einem auf 50 °C temperierten Ölbad im Ölpumpenvakuum bei etwa 10⁻² bis 10⁻³ Torr getrocknet.
Ausbeute: 18,7 g (93,5 % der Theorie).

| Elementaranalyse: | |
|---|---|
| | Sr |
| ber. | 19,29 |
| gef. | 20,1 |

### Schmelzverhalten: unmittelbar nach der Herstellung:

Sintern bei 140 °C, bilden einer weißlichen, trüben Schmelze bei 250 °C.

Nach 30 Tagen: Sintern bei 145 °C, bilden einer weißgelben, trüben Schmelze bei 240 °C.

### Beispiel 5

### Anwendung von Ba(TMHD)₂ zur Erzeugung Ba enthaltender Schichten

In eine CVD-Apparatur wurde zunächst gasförmiges Cu(TMHD)₂ in die Apparatur eingeleitet und auf einem Strontiumtitanatträger unter Bildung einer Schicht zersetzt, welche Kupfer im wesentlichen als Oxid enthielt. Anschließend wurde Y(TMHD)₃ thermisch unter Bildung einer Schicht zersetzt, welche Yttrium im wesentlichen als Oxid enthielt.

Schließlich wurde nach Beispiel 1 hergestelltes, erfindungsgemäßes Ba(TMHD)₂ verdampft und in die CVD-Apparatur eingeleitet. Es wurde bei der Zersetzung auf dem auf 600 °C erhitzten Träger eine Schicht abgeschieden, die das Barium im wesentlichen in Form des Oxids enthielt.

Anschließend wurde in sauerstoffhaltiger Atmosphäre getempert.

## Patentansprüche

1. Verdampfbare Verbindungen der allgemeinen Formel (I)
M(R)₂ (I)
worin M Calcium, Strontium oder Barium und R 2,6-Dimethyl-3,5-heptandionat, 2,2,6-Trimethyl-3,5-heptandionat oder 2,2,6,6-Tetramethyl-3,5-heptandionat bedeutet, dadurch gekennzeichnet, daß im ¹H-NMR-Spektrum ein Signal für Wasserprotonen nicht nachweisbar ist.

2. Verbindungen nach Anspruch 1, dadurch gekennzeichnet, daß R 2,2,6,6-Tetramethyl-3,5-heptandionat bedeutet.

3. Verbindungen nach Anspruch 1 oder 2, dadurch gekennzeichnet, daß M Barium bedeutet.

4. Gemische zur Anwendung bei MOCVD-(metal organic chemical vapour deposition-)Verfahren, dadurch gekennzeichnet, daß sie eine oder mehrere verdampfbare Verbindungen der allgemeinen Formel (I), nach einem der Ansprüche 1 bis 3, und eine oder mehrere verdampfbare organische Verbindungen anderer Metalle enthalten.

5. Gemische nach Anspruch 4, dadurch gekennzeichnet, daß sie verdampfbare Verbindungen der allgemeinen Formel (I) und eine oder mehrere verdampfbare organische Verbindungen von Yttrium und Kupfer enthalten.

6. Verfahren zur Herstellung von Verbindungen der allgemeinen Formel (I)
M(R)₂ (I)
worin M Calcium, Strontium oder Barium und R 2,6-Dimethyl-3,5-heptandionat, 2,2,6-Trimethyl-3,5-heptandionat oder 2,2,6,6-Tetramethyl-3,5-heptandionat bedeuten durch Umsetzen einer Verbindung des Metalles M in einem Lösungsmittel mit der Diketonverbindung 2,6-Dimethyl-3,5-heptandion, 2,2,6-Trimethyl-3,5-heptandion bzw. 2,2,6,6-Tetramethyl-3,5-heptandion und Isolieren der gebildeten Verbindung der Formel (I), dadurch gekennzeichnet, daß man eine Lösung oder Suspension der Verbindung des Metalles M in Wasser oder in einem wasserhaltigen, mit Wasser mischbaren Lösungsmittel, mit dem Diketon versetzt, das gebildete Produkt abtrennt und im Vakuum bei einer Temperatur von maximal 60 °C trocknet, bis im ¹H-NMR-Spektrum Wasserprotonen nicht mehr nachweisbar sind.

7. Verfahren nach Anspruch 6, dadurch gekennzeichnet, daß man als Verbindung des Metalles M ein Hydroxid oder Oxid des Metalls M verwendet.

8. Verfahren nach Anspruch 6 oder 7, dadurch gekennzeichnet, daß man die Verbindung des Metalles M und die Diketon-Verbindung im Molverhältnis von 1:2 bis 1:2,05 einsetzt.

9. Verfahren zur Abscheidung einer Erdalkalimetall enthaltenden Schicht auf einem Substrat durch Zersetzung einer Erdalkalimetall-Diketonat-Verbindung, dadurch gekennzeichnet, daß man als Erdalkalimetall-Diketonat-Verbindung eine Verbindung der allgemeinen Formel (I)
M(R)₂ (I)
worin M Calcium, Strontium oder Barium und R 2,6-Dimethyl-3,5-heptandionat, 2,2,6-Trimethyl-3,5-heptandionat oder 2,2,6,6-Tetramethyl-3,5-heptandionat bedeutet und in deren ¹H-NMR-Spektrum Wasserprotonen nicht nachweisbar sind, verwendet.

10. Verfahren nach Anspruch 9, dadurch gekennzeichnet, daß R 2,2,6,6-Tetramethyl-3,5-heptandionat bedeutet.

11. Verfahren nach Anspruch 9 oder 10, dadurch gekennzeichnet, daß M Barium bedeutet.

12. Verfahren nach einem der Ansprüche 9 bis 11, dadurch gekennzeichnet, daß man die Verbindung der allgemeinen Formel (I) in der Gas- oder Dampfphase zersetzt.

13. Verfahren nach Anspruch 12, dadurch gekennzeichnet, daß man die Verbindung der allgemeinen Formel (I) unter vermindertem Druck, gewünschtenfalls unter Anwendung eines Trägergases, in die Gas- oder Dampfphase überführt und bei vermindertem Druck zersetzt.

14. Verfahren nach einem der Ansprüche 9 bis 13, dadurch gekennzeichnet, daß man zur Abscheidung von Erdalkalimetall enthaltenden Schichten, welche das Erdalkalimetall in Form von Erdalkalimetalloxid, Erdalkalimetallhydroxid oder Erdalkalimetallcarbonat enthalten, eine Nachbehandlung in einer reaktiven Atmosphäre, vorzugsweise einer oxidierenden, hydrolysierenden und/oder carbonisierenden Gasatmosphäre durchführt.

15. Verfahren nach einem der vorhergehenden Ansprüche, dadurch gekennzeichnet, daß man zur Abscheidung von Erdalkalimetall enthaltenden Schichten, welche weiterhin ein anderes Metall oder mehrere andere Metalle enthalten, eine oder mehrere Verbindungen anderer Metalle und eine Verbindung der allgemeinen Formel (I) gleichzeitig oder nacheinander zersetzt.

## Claims

1. Vaporisable compounds of the general formula (I),
M(R)₂ (I)
wherein M is calcium, strontium or barium and R is 2,6-dimethyl-3,5-heptane dionate, 2,2,6-trimethyl-3,5-heptane dionate or 2,2,6,6-tetramethyl-3,5-heptane dionate, characterised in that a signal for water protons cannot be detected in the ¹H-NMR spectrum.

2. Compounds according to Claim 1, characterised in that R is 2,2,6,6-tetramethyl-3,5-heptane dionate.

3. Compounds according to Claim 1 or 2, characterised in that M is barium.

4. Mixtures for use in MOCVD (metal organic chemical vapour deposition) processes, characterised in that they contain one or more vaporisable compounds of the general formula (I) according to one of Claims 1 to 3, and one or more vaporisable organic compounds of other metals.

5. Mixtures according to Claim 4, characterised in that they contain vaporisable compounds of the general formula (I) and one or more vaporisable organic compounds of yttrium and copper.

6. A process for preparing compounds of the general formula (I),
M(R)₂ (I)
wherein M is calcium, strontium or barium and R is 2,6-dimethyl-3,5-heptane dionate, 2,2,6-trimethyl-3,5-heptane dionate or 2,2,6,6-tetramethyl-3,5-heptane dionate, by reacting a compound of the metal M in a solvent with the diketone compound 2,6-dimethyl-3,5-heptane dione, 2,2,6-trimethyl-3,5-heptane dione or 2,2,6,6-tetramethyl-3,5-heptane dione and isolating the resulting compound of Formula (I), characterised in that a solution or suspension of the compound of the metal M in water or in a water-containing solvent which is miscible with water is mixed with the diketone, the resulting product is separated off and dried in a vacuum at a temperature of at most 60°C, until water protons can no longer be detected in the ¹H-NMR spectrum.

7. A process according to Claim 6, characterised in that a hydroxide or oxide of the metal M is used as the compound of the metal M.

8. A process according to Claim 6 or 7, characterised in that the compound of the metal M and the diketone compound are used in a molar ratio of 1:2 to 1:2.05.

9. A process for depositing a layer containing alkaline earth metal on a substrate by decomposing an alkaline earth metal-diketonate compound, characterised in that a compound of the general formula (I),
M(R)₂ (I)
wherein M is calcium, strontium or barium and R is 2,6-dimethyl-3,5-heptane dionate, 2,2,6-trimethyl-3,5-heptane dionate or 2,2,6,6-tetramethyl-3,5-heptane dionate and in the ¹H-NMR spectrum of which water protons cannot be detected, is used as the alkaline earth metal-diketonate compound.

10. A process according to Claim 9, characterised in that R is 2,2,6,6-tetramethyl-3,5-heptane dionate.

11. A process according to Claim 9 or 10, characterised in that M is barium.

12. A process according to one of Claims 9 to 11, characterised in that the compound of the general formula (I) is decomposed in the gas or vapour phase.

13. A process according to Claim 12, characterised in that the compound of the general formula (I) is converted into the gas or vapour phase under reduced pressure, if desired using a carrier gas, and is decomposed at reduced pressure.

14. A process according to one of Claims 9 to 13, characterised in that for depositing layers containing alkaline earth metal which contain the alkaline earth metal in the form of alkaline earth metal oxide, alkaline earth metal hydroxide or alkaline earth metal carbonate, subsequent treatment in a reactive atmosphere, preferably an oxidising, hydrolysing and/or carbonising gas atmosphere, is carried out.

15. A process according to one of the preceding Claims, characterised in that for depositing layers containing alkaline earth metal which furthermore contain another metal or a plurality of other metals, one or more compounds of other metals and a compound of the general formula (I) are decomposed at the same time or successively.

## Revendications

1. Composés vaporisables de formule générale (I)
M(R)₂ (I)
dans laquelle M est le calcium, le strontium ou le baryum, et R représente le 2,6-diméthyl-3,5-heptanedionate, le 2,2,6-triméthyl-3,5-heptanedionate ou le 2,2,6,6-tétraméthyl-3,5-heptanedionate, caractérisés en ce qu'aucun signal de protons de l'eau ne peut être décelé dans le spectre RMN-¹H.

2. Composés selon la revendication 1, caractérisés en ce que R est le 2,2,6,6-tétraméthyl-3,5-heptanedionate.

3. Composés selon la revendication 1 ou 2, caractérisés en ce que M est le baryum.

4. Mélanges destinés à être utilisés dans un procédé MOCVD (déposition d'un composé organométallique en phase vapeur), caractérisés en ce qu'ils contiennent un ou plusieurs composés vaporisables de formule générale (I) selon une des revendications 1 à 3, et un ou plusieurs composés organiques vaporisables d'autres métaux.

5. Mélanges selon la revendication 4, caractérisés en ce qu'ils contiennent des composés vaporisables de formule générale (I) et un ou plusieurs composés organiques vaporisables de l'yttrium et du cuivre.

6. Procédé pour préparer des composés de formule générale (I)
M(R)₂ (I)
dans laquelle M est le calcium, le strontium ou le baryum, et R est le 2,6-diméthyl-3,5-heptanedionate, le 2,2,6-triméthyl-3,5-heptanedionate ou le 2,2,6,6-tétraméthyl-3,5-heptanedionate, par réaction d'un composé du métal M dans un solvant avec la dicétone 2,6-diméthyl-3,5-heptanedione, 2,2,6-triméthyl-3,5-heptanedione ou 2,2,6,6-tétraméthyl-3,5-heptanedione, et isolement du composé de formule (I) ainsi formé, caractérisé en ce qu'on ajoute la dicétone à une solution ou une suspension du composé du métal M dans l'eau ou dans un solvant aqueux miscible à l'eau, on isole le produit formé, et on le sèche sous vide à une température maximale de 60°C jusqu'à ce qu'on ne puisse plus déceler de protons de l'eau sur le spectre RMN-¹H.

7. Procédé selon la revendication 6, caractérisé en ce qu'on utilise comme composé du métal M un hydroxyde ou un oxyde du métal M.

8. Procédé selon la revendication 6 ou 7, caractérisé en ce qu'on utilise le composé du métal M et la dicétone selon une proportion molaire de 1:2 à 1:2,05.

9. Procédé pour séparer une couche contenant un métal alcalino-terreux à partir d'un substrat, par décomposition d'un dicétonate d'un métal alcalinoterreux, caractérisé en ce qu'on utilise comme dicétonate d'un métal alcalino-terreux un composé de formule générale (I)
M(R)₂ (I)
dans laquelle M est le calcium, le strontium ou le baryum, et R est le 2,6-diméthyl-3,5-heptanedionate, le 2,2,6-triméthyl-3,5-heptanedionate ou le 2,2,6,6-tétraméthyl-3,5-heptanedionate, et dans le spectre RMN-¹H duquel on ne peut déceler de protons de l'eau.

10. Procédé selon la revendication 9, caractérisé en ce que R est le 2,2,6,6-tétraméthyl-3,5-heptanedionate.

11. Procédé selon la revendication 9 ou 10, caractérisé en ce que M est le baryum.

12. Procédé selon l'une des revendications 9 à 11, caractérisé en ce que le composé de formule générale (I) est décomposé en phase gazeuse ou en phase vapeur.

13. Procédé selon la revendication 12, caractérisé en ce qu'on fait passer en phase gazeuse ou en phase vapeur le composé de formule générale (I) sous pression réduite, éventuellement par utilisation d'un gaz porteur, et qu'on le décompose sous pression réduite.

14. Procédé selon l'une des revendications 9 à 13, caractérisé en ce que, pour séparer des couches contenant un métal alcalino-terreux, qui contiennent le métal alcalino-terreux sous forme d'un oxyde d'un métal alcalino-terreux, d'un hydroxyde d'un métal alcalinoterreux ou d'un carbonate d'un métal alcalino-terreux, on procède à un post-traitement dans une atmosphère réactive, de préférence dans une atmosphère gazeuse oxydante, hydrolysante et/ou carbonisante.

15. Procédé selon l'une des revendications précédentes, caractérisé en ce que, pour séparer des couches contenant un métal alcalino-terreux et contenant en outre un autre métal ou plusieurs autres métaux, on décompose simultanément ou successivement un ou plusieurs composés d'autres métaux et un composé de formule générale (I).
